# EUROPEAN PATENT APPLICATION

(11) **EP 4 459 631 A1**
(43) Date of publication of application: **06.11.2024**
(21) Application number: 23171399.1
(22) Date of filing: 03.05.2023
(51) Int. Cl.: G16H 40/20

(54) **TRANSFER OF MEDICAL PROTOCOL STEP**

(71) Applicant: Koninklijke Philips N.V., 5656 AG Eindhoven (NL)
(72) Inventor: BOUWMAN, Wilbert Hendrik, 5656 AG Eindhoven (NL); BULUT, Murtaza, 5656 AG Eindhoven (NL); RASMIJN, Anita, 5656 AG Eindhoven (NL)
(74) Representative: Philips Intellectual Property & Standards

(57) **Abstract**

A computer-implemented method for requesting a protocol step to be performed at a different medical location. The method comprises identifying, at a first location, a first protocol step to be performed. Based on identifying the first protocol step to be performed, the method further comprises identifying a second location with a second protocol step and comparing the first protocol step and the second protocol step to determine whether a first output from performing the first protocol step would be comparable to a second output from performing the second protocol step. Based on the comparison, the second location is requested to perform the second protocol step.

## Description

### FIELD OF THE INVENTION

The invention relates to the transfer of medical protocol steps between medical locations.

### BACKGROUND OF THE INVENTION

Clinical practice guidelines (CPGs) are systematically developed statements to assist practitioner and patient decisions about appropriate health care for specific clinical circumstances. CPGs aim to promote evidence-based delivery of healthcare, reduce inappropriate variations in practice and reduce or limit costs of therapies or procedures.

The CPGs contain recommendations that are based on evidence from clinical trials, a rigorous systematic review and published medical literature.

Hospital protocols focus on procedural or operational details and are documents or SOPs (standard operation procedures) that describe how procedures are conducted and CPGs are applied. Hospitals define their protocols based on these CPGs recommendations.

Hospital protocol procedures/tasks are usually carried out in one hospital, but they could also be carried out by other healthcare providers. For investigation of a certain disease, a patient may visit different health care providers (e.g., radiologists, general practitioners, etc.). For example, after initial diagnosis, the first healthcare professional (HCP) could refer the patient to a more appropriate expert in another hospital, the patient may want to get different opinions from different HCPs or there may be a high burden of hospital, e.g., during the COVID pandemic.

Hospital protocol execution often takes place in one hospital, although in some cases patients are referred to another hospital in case of high hospital burden or to a more specialized hospital. Patient data exchange between hospitals is possible, but often, especially for imaging, measurements may need to be repeated to match vendor specific measurement devices or hospital specific imaging protocols.

In particular, acquisition of medical images is standardized to a rather low degree. Images acquired by different HCPs can differ significantly due to application of different acquisition parameters, different scanners from different manufacturers, or even different scanner versions from the same manufacturer. These differences cause many HCPs to repeat the same scan (previously acquired by another HCP) on their own machine to obtain images of a type which they are used to/have been trained on. By repeating measurements, data and quality will match hospital specific protocols and workflows. However, the repetition of measurements, or protocol steps in general, requires further time and resources to accomplish.

Thus, there is a need for an improved framework which can improve the transfer of medical protocol steps between locations.

### SUMMARY OF THE INVENTION

The invention is defined by the claims.

According to examples in accordance with an aspect of the invention, there is provided a computer-implemented method for requesting a protocol step to be performed at a different medical location, the method comprising:
identifying, at a first location, a first protocol step to be performed; and
based on identifying the first protocol step to be performed:
   identifying a second location with a second protocol step;
   comparing the first protocol step and the second protocol step to determine whether a first output from performing the first protocol step would be comparable to a second output from performing the second protocol step; and
   based on the comparison, requesting the second location to perform the second protocol step.

A protocol step is a step in a protocol (e.g., a hospital protocol) which provides guidance on how to perform a medical task. For example, a protocol step may indicate that a measurement is to be made, that data is to be collected, that data is to be analyzed etc. A protocol step provides guidance on how to perform a medical task. A protocol may provide guidance on a series of tasks (i.e., protocol steps) to be performed on the subject (e.g., for a suspected or identified pathology). In some cases, a protocol step can refer to standard operating procedures, and in some cases protocol steps can be referred to as procedures.

Different protocol steps for the same task may lead to outputs which are not comparable. In these cases, the staff at the first location may not understand and/or trust the output of the protocol step which can lead to a disruption in the execution of the protocol as the task now has to be repeated at the first location.

Additionally, protocol steps are often changed or updated in medical locations. Thus, comparing the protocol steps based on identifying the first protocol step to be performed ensures that the second output will be comparable prior to the second protocol step being performed.

In an embodiment, when the first output is determined to be comparable to the second output, the second location can be requested to perform the second protocol step without modification of the second protocol step.

Whether the first and second outputs are comparable is generally based on whether the protocol step has to be repeated at the first location. When the first and second outputs are comparable, this ensures continuity of care as the protocol step does not have to be repeated. The skilled person would be readily capable of determining when the first and second outputs are comparable.

For example, in imaging measurements (e.g., computed tomography (CT) scans, magnetic resonance imaging (MRI) etc.), properties such as the visualization of the image (e.g., gray scale or color, gamma correction, voxel interpolation, postprocessing filters etc.) can determine whether the measurements will be comparable. Image contrast properties can also determine whether the measurements will be comparable. Similarly, geometric properties (e.g., angulation, orientation, spatial resolution, spatial fidelity etc.) can also determine whether the measurements will be comparable.

A comparable second output does not have to be identical to the first output which would be obtained at the first location. A comparable output should, however, be able to be compared by a relevant clinician to an output obtained using the first location. As such, the second output can be used for its intended purpose and there is no need to repeat the task.

The method may be for requesting one or more protocol steps to be performed at a different location.

Requesting the second location to perform the second protocol step may comprise, based on the first output being determined to not be comparable to the second output, modifying the second protocol step based on the first protocol step to generate a modified protocol step, such that the first output would be comparable to a third output of the modified protocol step, and requesting the second location to perform the modified protocol step.

In an embodiment, if the outputs are determined not to be comparable, the second protocol step can be adapted/modified, and the second location can be requested to use the modified protocol step. This ensures the third output is comparable to an output which could be obtained by the first location. Thus, clinicians at the first location can understand and trust the third output.

The second protocol step may be modified at the first location, at the second location or at third, distinct, location (e.g., a central location that manages the transfer between locations).

Comparing the first protocol step and the second protocol step may comprise identifying a plurality of comparable protocol steps, wherein an output of each of the comparable protocol steps is comparable to the first output and comparing the second protocol steps and the comparable protocol steps.

This provides a list of protocol steps, each ensuring that a comparable output to that of the first protocol step can be obtained. Thus, the second protocol step can be compared to the comparable protocol steps instead of directly comparing it to the first protocol step.

The comparable protocol steps may each specify a medical device and/or medical software and corresponding settings. The comparable protocol steps may also, or alternatively, specify a particular method for performing the comparable protocol step.

For example, a list of comparable medical devices, and corresponding comparable parameters, which are capable of performing a comparable measurement to that of the first location may be provided. The comparable parameters, when used on the corresponding comparable medical device, ensure that the measurement is a comparable measurement.

Modifying the second protocol step may comprise adapting the second protocol step based on the one or more comparable protocol steps.

When the second protocol step has to be modified, it can just be adapted based on the one or more comparable protocols steps which are known to provide a comparable output.

The second protocol step may be adapted to be comparable or identical to one of the comparable protocol steps.

Identifying the plurality of comparable protocol steps may comprise identifying a plurality of medical resources at different medical locations capable of generating an output related to the first protocol step, obtaining parameters for the identified medical resources, determining whether the identified medical resources, using the corresponding parameters, are capable of generating an output comparable to the first output and identifying comparable protocol steps, each indicating a medical resource and the corresponding parameters, based on the identified medical resources and the corresponding parameters which are capable of generating an output comparable to the first output.

In a first example, the medical resource is a medical device and/or medical software and the parameters comprise corresponding settings for the medical device and/or medical software.

Identifying the plurality of comparable protocol steps may further comprise determining a capacity of the different medical locations corresponding to the identified medical resources, wherein identifying comparable protocol steps is further based on the capacity of the different medical locations.

The capacity of the different medical locations may include the availability of staff capable of performing the protocol steps and/or the availability of medical resources in the corresponding locations required for the protocol steps.

This can ensure that the list of comparable protocol steps (i.e., the augmented protocol step) only contains comparable protocol steps at medical locations with the capacity of performing the protocol step.

The first protocol step may indicate a first medical resource capable of generating the first output and one or more first parameters of the first medical resource suitable for generating the first output. The second protocol step may indicate a second medical resource capable of generating the second output and one or more second parameters of the second medical resource. Comparing the first protocol step and the second protocol may comprise comparing the first medical resource and the second medical resources and comparing the first parameters and the second parameters.

This provides a workflow between medical locations (e.g., different medical facilities) which considers which medical resources (e.g., medical devices and/or software used) are used for the first output and the parameters which are used thereon such that the second output obtained at the second location (e.g., a different hospital or a different department of a same hospital) is comparable to the outputs typically obtained at the first location.

For example, some types of medical devices may not be capable of obtaining measurements which are comparable to measurements from other medical devices. Similarly, even if two different medical devices can obtain comparable measurements, the parameters used for the measurements should be set such that the measurements are comparable.

This reduces the probability that the outputs obtained at different locations have to be repeated because they are not comparable. As such, the subject's experience and/or safety is improved due to, for example, fewer measurements (e.g., lower doses of radiation) and the burden and cost on the medical locations is reduced as there will be less repetition, leading to an improvement to the efficiency of the medical locations.

The parameters may include settings for a corresponding medical device and/or settings for the software used (e.g., setting confidence intervals for the outputs). The parameters can directly affect properties of the outputs. In order for outputs from different locations to be comparable, the properties of the measurements should be as similar as possible to ensure non-disturbed execution of the protocol (i.e., without the need to repeat the protocol steps). As such, it is preferable that the second medical resource generates second outputs with similar properties to the first output obtained with the first medical resource using the corresponding properties. The extent to which the outputs are similar can be determined by the skilled person.

The first and second parameters are generally the parameters preferred/used by the corresponding medical location.

It is preferable that the subject is only transferred to the second medical location if it is capable of performing a comparable measurement.

Of course, this is only necessary if it is not yet known whether the second medical location is capable of a comparable measurement. In some cases, it may already be predetermined whether the second medical location is capable. For example, there may exist a database showing which medical locations are capable of comparable measurements (e.g., they may have the same device). The database may be based on previous measurements. In the case where the second location is known to be capable, it is possible to only have to modify the parameters used at the second location to ensure a comparable output/measurement.

The first and second outputs may be first and second measurements of the subject respectively. The first and second medical resources may be first and second medical devices respectively.

The method may further comprise determining a capacity of the first location and/or second location, wherein requesting the second location to perform the second protocol step is based on the capacity of the first location and/or the second location.

It may be preferable to only transfer the subject if the capacity of the first/second location is above a first/second capacity threshold.

The capacity of the first location and/or the second location may be at least partly defined by one or more of the availability of staff capable of performing the first protocol step or the second protocol step respectively and the availability of medical resources required for the first protocol step or the second protocol step respectively.

Some protocol steps may require resources (e.g., software or equipment such as scanners, contrast agent, medication etc.). As such, it may be preferable to check that such resources are available. The resources may comprise equipment which generally includes any relevant medical devices, if applicable.

It may be preferable to check the availability of staff in the second location capable of performing the second or modified protocol step and/or the availability of equipment in the second location required for the second or modified protocol step.

The first and second outputs may be imaging measurements of a subject.

Imaging measurements (i.e., obtaining a medical image of the subject) are not very well standardized and thus it is common to see the properties of imaging measurements differ significantly between medical locations. This often results in imaging measurements from different medical locations not being comparable. This can lead to repetition of the imaging measurements when subjects are transferred between medical locations. The first output would be a first medical image and the second output would be a second medical image.

The method may further comprise imaging the subject, using the second protocol step, at the second location.

When the second protocol step is modified, the method may alternatively comprise imaging the subject using the modified protocol step at the second location.

The method may further comprise adapting one or more later, or previous, protocol steps to be performed by the first location based on the second output of the second protocol step.

The invention also provides a computer program carrier comprising computer program code which, when executed on a processing system, causes the processing system to perform all of the steps of any of the afore-mentioned methods.

The computer program carrier may be a data storage device (e.g., solid-state drive, hard drive etc.) The computer program carrier may be bitstream.

The invention also provides a system for requesting a protocol step to be performed at a different medical location, the system comprising a processor configured to:
identify, at a first location, a first protocol step to be performed; and
based on identifying the first protocol step to be performed:
   identify a second location with a second protocol step;
   compare the first protocol step and the second protocol step to determine whether a first output from performing the first protocol step would be comparable to a second output from performing the second protocol step; and
   based on the comparison, request the second location to perform the second protocol step.

The processor may be configured to request the second location to perform the second protocol step by, based on the first output being determined to not be comparable to the second output, modifying the second protocol step based on the first protocol step to generate a modified protocol step, such that the first output would be comparable to a third output of the modified protocol step, and requesting the second location to perform the modified protocol step.

The processor may also be configured to perform any of the steps of the afore-mentioned method for requesting a protocol step to be performed at a different medical location.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows the generation of an augmented protocol;
Fig. 2 shows the generation of a modified protocol step;
Fig. 3 shows the generation of scheduling and staffing at a second location;
Fig. 4 shows a flow chart for requesting the transfer of a protocol step; and
Fig. 5 shows a method for requesting a protocol step to be performed at a different medical location.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a computer-implemented method for requesting a protocol step to be performed at a different medical location. The method comprises identifying, at a first location, a first protocol step to be performed. Based on identifying the first protocol step to be performed, the method further comprises identifying a second location with a second protocol step and comparing the first protocol step and the second protocol step to determine whether a first output from performing the first protocol step would be comparable to a second output from performing the second protocol step. Based on the comparison, the second location is requested to perform the second protocol step.

A protocol step is a step in a protocol which specifies how to perform a medical task (e.g., a procedure). For example, a protocol step may specify how a measurement of a subject is performed. A protocol step may alternatively specify how to perform other medical tasks (e.g., treatment decision, treatment administration, medication administration, data acquisition, data analysis etc.).

A second output is comparable to the first output if it eliminates the need to repeat the first protocol step because the second output cannot be used as expected. This may be based on properties of the output which a clinician (or other relevant expert) is used to and/or properties of the output which match the specifications, standards, and/or guidelines defined by the corresponding organization (e.g., hospital-specified guidelines). For example, in imaging scans, properties of the scan itself can be used to determine if the scans would be comparable.

Previous outputs obtained at the first location by performing the first protocol step can be compared to previous outputs obtained at the second location by performing the second protocol steps to determine if the outputs are comparable.

Alternatively, or additionally, pre-determined specifications of resources (e.g., software, scanners, medical devices etc.) can be used to determine/calculate if the outputs would be comparable. Comparability may be checked by comparing properties of the output to pre-determined requirements. If comparability cannot be determined via the comparison to the pre-determined requirements, comparability can be flexibly calculated on-demand.

Two different types of comparisons can be considered. The first type is the comparison of objective data and information which can be generated by devices or software. Examples of this type of output include images, signals, lab results, visualizations of data, and computed scores. Naturally the comparison of different types of outputs depends on the output type and characteristics.

Features calculated directly from the output, such as frequency analysis, signal to noise ratio, missing data, confidence measures etc. can be considered as objective data. There exist many different features which can be calculated from the outputs and can be determined by the corresponding locations.

Features relating to the device, software, or processes that were used to generate the corresponding outputs can also be considered as objective data. In other words, the comparability between the settings of the devices, software and/or process steps can be determined during the comparison. Typically, such settings will be stored in log files.

To calculate the comparability, different similarity or distance measures (between the selected output features) can be used to determine a comparability score. For example, distance measures include Euclidean, Manhattan, Chebyshev, Minkowski, Cosine, Pearson, Mahalanobis, Squared Eucliedean, Jaccard, Levenshtein, Sorensen-Dice, Jensen-Shannon, Canberra, Hamming, Spearman, Chi-Square, Tanimoto distances, correlations, entropy, and Kolmogorov-Smirnov test computations. In other words, the goal is to compare the features of two outputs.

The second type is a comparison of subjective data or information generated with human input or effort. Examples include interpretation of patient data, interpretation of lab results and clinical notes. To calculate the comparability between these, the qualifications and experience of people involved in generation of these results can be compared.

If the partner locations (e.g., hospitals) is selected according to a particular pre-determined standard, the first type of the comparisons using objective data is preferably used for the comparison (i.e., based on the low-level features extracted from the outputs and/or device and software setting from, for example, settings extracted from log files).

For both types of comparison, not only the output comparability, but also comparability of the means to produce the output can be considered. In other words, the actions performed prior to output generation, can be also included in the comparability calculations. This is to ensure that the standard of practice at the first location matches the standard of practice at the second location.

To assess the means to produce the output, a score relating to how the corresponding protocols in the corresponding locations were followed in practice can be calculated. In other words, the actual realization of a set of protocol steps (i.e., what has been really done) can be compared to the suggested realization (i.e., what is formally described) and, based on this, a score can be computed. If the actual realization matches (within certain limits) the suggested realization of procedures prior to generating the output, then it can be assumed that a standard of practice has been followed and the output can be labeled as trusted.

Another aspect of comparability relates to the comparison of augmented protocols. The protocol steps can be augmented with different types of labels, and meta information. In other words, the augmentation can be considered as a process of adding additional description for a given protocol steps. These descriptions can be in terms of devices, output requirements, process requirements, expertise requirements, links to prior or following steps, specific instruction, links to relevant documentations, etc.

Calculating a comparability score between two protocol steps can be achieved by comparing the protocol steps and the additional information attached to these steps. In an example, the protocol steps may require the usage of a particular device usage and the additional information can relate to device settings, where the device settings can be used as a base for protocol comparisons.

In a more general sense, all descriptors attached to a protocol step can be used to compare it with another protocol steps, which will also include set of descriptors. In most cases, these descriptors will be text based, and therefore natural language processing algorithms can be used to determine the similarity or dissimilarity between two protocol steps. In an example, a specific type of data, such as example images, signals, lab results etc., is attached to the protocol steps as metadata or descriptors. Algorithms are used to compare these attached objects to determine the similarity between two protocol steps. For example, a first image linked to the first protocol step from the first location can be compared to a second image linked to the second protocol step from the second location. The results of the comparisons can be used to determine the match or mismatch (i.e., the comparability) between protocol steps.

An augmented protocol can be generated by, for example, considering devices, methods etc. specified in a protocol to determine the link between the vendor information of the device and output specification. The labelling can be for hardware and/or software.

Information (of a device or a protocol) can be extracted from specifications (pre-written information) and/or and from the historical data collected from device and protocol use. Device usage can be determined through device log file, and protocol application can be determined by EMR records.

The augmented protocol specifies, per protocol step, per measurement, different options for performing the measurement using different hardware and software alternatives. In addition, workflow information indicating the duration and number and expertise of staff needed can be included.

Using the augmented protocols, devices and protocols from different locations can now be compared (i.e., similarity scores or different distance measures calculated) and ranked on comparability. A typical scale for comparability scores will be from 0 to 1, where 0 indicates no comparability, and 1 indicates the best possible comparability. The definitions of boundaries (0 and 1) can be determined by the institutions, as well as the definitions of the intermediate numbers between 0 and 1.

The locations may include medical facilities such as hospitals or other healthcare-related facilities. The first and second locations may be different departments of the same medical facility.

This invention relates to clinical decision support (CDS). More specifically, the invention addresses workflow support to optimize the delivery of health services.

To match workflows and protocols (or parts of the workflows/protocols) between hospitals (or other healthcare facilities), a protocol transformation/modification/augmentation would be desired which considers measurement devices and/or measurement methods and potential hospital referral. This solution may further lead to lower burden of hospitals, hospital staff and improve patient experience.

Parts of the activities/steps required by the hospital protocols can be transferred to other locations to lower the burden of the hospital and to facilitate the needs and adherence to clinical guidelines of the original hospital. Transferring protocol steps could include hospital to hospital, transferring from one department to another department and could also include transferring from hospital to a decentralized facility, such as a dedicated imaging facility. These decentralized facilities are also called outpatient clinics.

For example, during cancer treatment, imaging methods are often used to monitor treatment efficiency and disease relapse. These imaging procedures could be performed by the hospital, but a decentralized imaging center could also perform these imaging assessments. When protocol and device specifications, including software settings, are known from the hospital, a similar scan and assessment can be performed by such a center. These centers can serve as a center for all hospitals in which the center has measurement devices from multiple vendors to enable hospital specific guideline adherence and data comparison.

In times when demand exceeds capacity, there is a need for hospital-to-hospital data collection solution without compromising on healthcare requirements set by hospital protocols, to lower the pressure in hospitals.

It is proposed to assess and determine the hospital measurement (data collection) capabilities and options from one hospital that match hospital protocol requirements and hospital measurements of the second hospital and use this information to enable protocol adaptations/modifications/augmentation, for example, by dynamically assigning the measurement devices.

A system is proposed that suggests if a step in the protocol can be performed in multiple hospitals or multiple departments based on vendor and output, indicates what the constrains are when a protocol step is transformed and transferred to the second hospital and, if required, adapts the protocol of the second hospital to match the first hospital.

This can improve the patient experience and safety due to, for example, fewer measurements (e.g., reduced radiation exposure in case of CT). This solution can also lower the burden to hospital and staff caused by the redundancy of repeating measurements. Costs to hospitals (e.g., due to less imaging) can also be lowered and efficiency can also be improved by the transformation of protocol steps, or parts of the protocol (i.e., multiple protocol steps) between hospitals.

A protocol transformation solution is proposed in which the system determines if a hospital protocol task can be transformed towards a task in a second hospital using specified criteria. Hospital protocols and used measurement devices/methods, including specs from various locations (e.g., hospitals, medical facilities etc.), the burden of the hospitals (e.g., capacity/demand ratio) and the device and staff scheduling can be used as inputs.

A main aspect of the proposed system is to use a protocol augmented with additional options for measurement devices, software and/or methods. When required, the capacity or demand of the different locations can be considered. If it is determined that there is an option to transfer/transform/modify the protocol, device and staff scheduling can also be considered.

The system can use these inputs to label protocols with the requirements related towards devices and methods, as well as staff and expertise needed, to match and rank hospitals which could do a comparable measurement which can be used by the requesting (first) hospital and schedule devices and expert staff based on the matching hospital and device.

The expertise needed generally refers to staff expertise to perform the protocol step. Staff expertise can include education level, compliance to regulations and/or experience.

Measurement device/software/method suggestion per hospitals, adaptations of the protocol step to enable the output and adapted scheduling related to staff and devices can be output by the system accordingly.

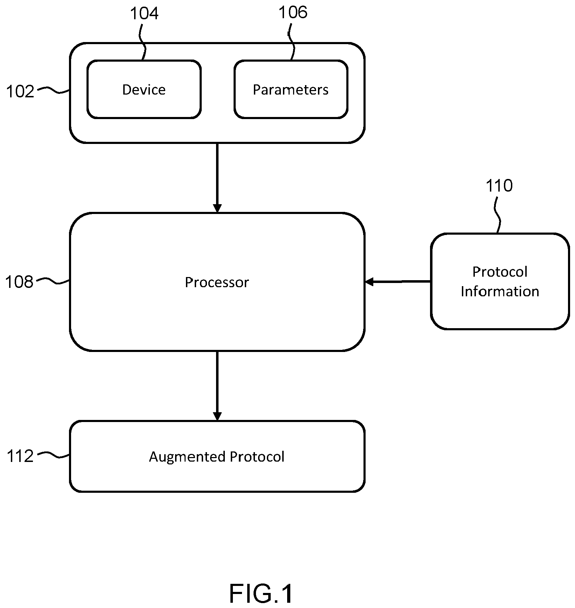

Fig. 1 shows the generation of an augmented protocol 112. The augmented protocol 112 includes a list of comparable protocol steps (e.g., including corresponding devices, software, methods, locations, expertise, staffing, resources etc.), where the output of the comparable protocol steps is comparable to the output of the first protocol step 102 at a first facility. The first protocol step 102 may specify a device 104 (or other resource, such as relevant software) and corresponding parameters 106 to perform a particular medical task. The first protocol step 102 is typically performed at the first facility to obtain a first output (e.g., a measurement, analysis etc.). The first protocol step 102 is input into a processor 108 together with protocol information 110. The protocol information 110 may include information on the first output as well as information on other relevant devices and parameters. The protocol information 110 can be used by the processor 108 to identify and determine the comparable protocol steps which, together, form the augmented protocol 112.

In times where care demand is higher than hospital capacity, transformation of protocol steps would be desired to lower the pressure of the hospital. Hospitals can often use multiple measurement devices for the same goal, for example multiple CT, MRI scanners from different vendors are available within a hospital. Based on scheduling, protocols and experience on how the measurements are used by the experts, hospitals can select a certain device for the measurement step.

This advantage can be used to determine, in the case of high burden, if protocol steps can be performed by another hospital (or location) and modify (transform) the protocol step accordingly. In some cases, more than one step of a protocol may be modified.

Protocol information 110 (e.g., vendor information, device usage etc.) and an output specification (e.g., determined from the first protocol step 102) can be used by the processor 108 to determine the link between, for example, vendor information and the output specification. The inputs can include both hardware (i.e., the device 104) or software.

Protocol information can be extracted from the specifications (pre-written information), and/or from the historical data collected from device and protocol use. Device usage can be determined through device log file, and protocol application can be determined by EMR records.

The output of the processor 108 is an augmented protocol 112 where, per protocol step, different options for obtaining the corresponding output using different hardware and software alternatives, and/or different methods, are indicated. In addition, workflow information indicating the duration and number and expertise of staff needed can be included.

In some cases, the protocol steps of a second location, to which the protocol step is transferred, may need to be modified.

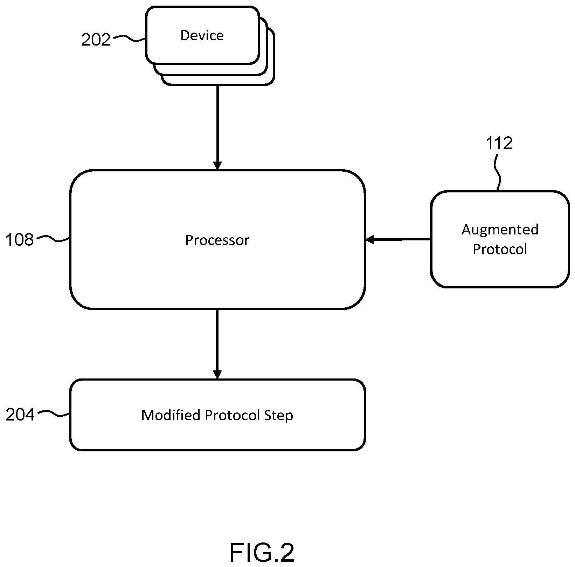

Fig. 2 shows the generation of a modified protocol step 204. In this case, information related to a plurality of devices 202 and the augmented protocol 112 are used by the processor 108 to determine if, for example, a measurement device in the first location (e.g., a first hospital) matches the available devices used in the second location (e.g., a second hospital).

The matched device can then be included in the modified protocol step 204. In this example, only devices 202 from a second location (e.g., the second hospital) are used for matching. However, ideally, multiple locations can be included in the search, in which case, based on ranking of similarities in protocol steps between the locations, the best match will be shown first.

Note that, in the aforementioned examples, a single augmented protocol 112 is used. However, each location may also have an augmented protocol and multiple augmented protocols from different locations can be used by the processor 108 to determine the modified protocol step. It is also possible to use an augmented protocol, for example, for the second location, and non-augmented (typical) protocol from the first location hospital because the main goal is to aid the first location's operations by suggesting them to be performed somewhere else.

Where devices are referred to above, general resources can also be used which includes hardware, software, methods and expertise needed. Device information in the protocol information can include pre-specified descriptions, as well as information extracted from the application/usage of the device and protocol in practice. Device information may also include information on the device settings, device usage, instructions/methods and/or device outputs.

In an example, the specified imaging devices can be used to assess the similarity (i.e., the comparability) between the two imaging protocol steps. To determine options for transferring the protocol step from one location to another, specific parameters of the imaging devices can be used.

Image visualization can be used to determine comparability between protocol steps. Image visualization may be determined by, for example, the type of grey scale or type of color scale (e.g., rainbow, heated iron) used, gamma correction, type of voxel interpolation (spatially, sometimes also temporal) and postprocessing filters (e.g., denoising).

Image contrast can also be used to determine comparability between protocol steps. Image contrast may be determined by, for example, contrast from tissue properties (e.g., T1 and T2 in MR), contrast from acquisition parameters (e.g., TR and TE in MR) and artefacts from non-ideal acquisition procedure (e.g., B0 and B 1 inhomogeneities in MR or patient motion in general).

Geometric parameters can also be used to determine comparability between protocol steps. Geometric parameters may include, for example, angulation, orientation, spatial resolution and spatial fidelity/distortion,

Imaging devices could, among others, include magnetic resonance (MR), computed tomography (CT), diagnostic x-ray (DXR), ultrasound (US) and positron emission tomography (PET).

Augmenting the protocols in the second location to account for performing selected steps differently based on the availability of expert resources at the second location can include various aspects related to preparing devices, scheduling staff, and interpretation of results.

Protocol information which can be augmented or transformed could include staff requirements (number and skills), preparation steps (contrast agent, medication), procedures prior or after performing the protocol step, measurement software usage (settings), hardware parameters (imaging acquisition parameters), analysis of data (software guideline, parameter settings) and interpretation (methods and rules followed) of data.

As a part of the comparison of the protocol steps, the availability, expertise, and scheduling of the staff in the locations can be considered. Both hardware and software handling are dependent on experts available. In order to ensure the modified protocol step can be performed at the second location, the experts' availability can be taken into account and scheduled.

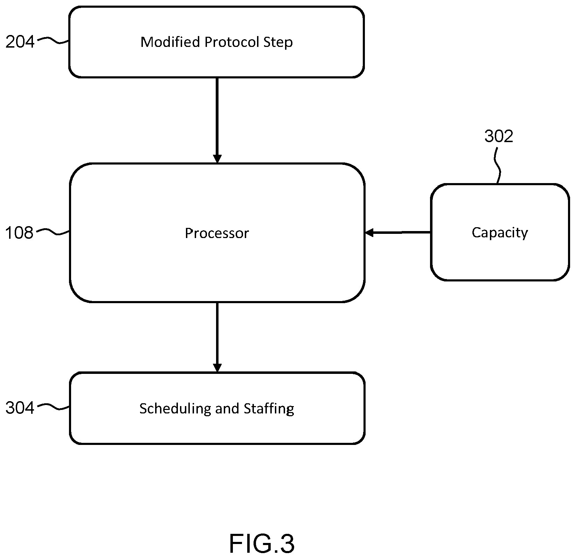

Fig. 3 shows the generation of scheduling and staffing 304 at the second location. The modified protocol 204 (which may include a device and staff specification) is now input into the processor 108 together the capacity 302 of the second location. The capacity 302 may include staff availability and device/resource scheduling. Based on this information, the staff and devices can be scheduled for the second location via scheduling and staffing 304.

If the modified protocol 204 includes a change of device or software usage, the staff and device scheduling may also be adapted at the first location for later protocol steps.

Depending on how the first protocol step is outsourced by the first location, and how it is performed at the second location, the protocol steps prior to or following the first protocol step may need to be modified.

For example, a protocol may indicate four protocol steps in the following order: A-B-C-D. Depending on expected steps and performance at the second location (which can be calculated from the augmented data + real time data from the second location), additional steps can be added or removed prior to a protocol step (e.g., if step C is transferred, protocol may be changed to indicate steps A-B-B'-C-D, or A-B-C-C'-D). The addition of a new step will typically be immediately before or after the transferred step C.

Alternatively, a step may be removed (e.g., steps A-C-D or A-B-C). Typically, steps immediately before or after the transferred steps will be removed. Another option is to change the order of the steps (e.g., A-C-B-D). This may be applicable when a step cannot be performed by the second location and has to be performed at the first location. The modification of the order may mean that the subject doesn't need to go back to the first location for said step and, instead, this step will be placed before or after the transferred steps.

A similar concept can be applied to the case where a protocol step has already been performed at the second location. Based on the generated output, at the second location, the way the output was generated and the modifications from a previous step (if they have been applied to the following steps), the following protocol step can be modified. The modifications will typically include adding, deleting, and/or changing the order of the steps as explained above.

Generally, it is preferable to adapt the prior steps based on the expected output and expected means to generate the output. To adapt the following steps, the actual data (the output, and the means it is generated) can be used. However, in some cases, it may be desirable to calculate potential adaptations for the following steps based on the expected information, as a means of planning.

In one example, a protocol step can be modified and transferred towards the second location. It might also be possible to transfer multiple steps or the entire protocol depending on the burden of the requesting location and effort needed to transform the protocol.

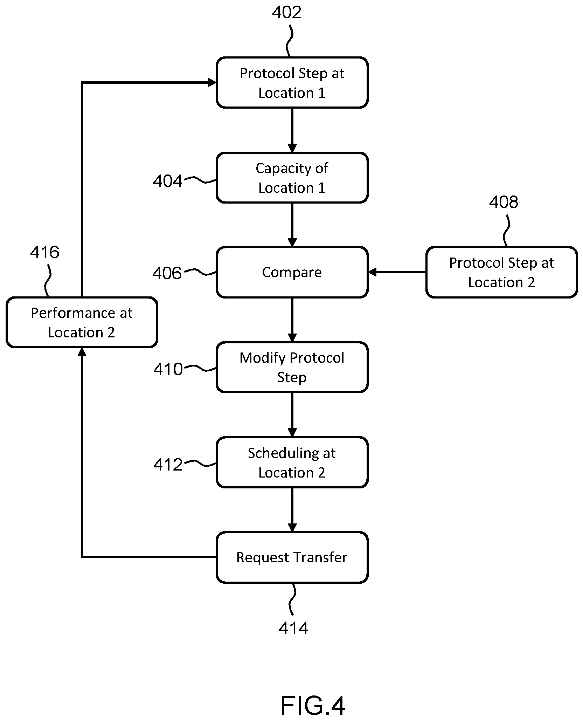

Fig. 4 shows a flow chart for requesting the transfer of a protocol step. In step 402, location 1 is following a first protocol step for a patient. The first protocol step may be part of a first protocol which consists out of five protocol steps. In step 404, the capacity of the first location is determined. If the demand of care is currently higher, or predicted to become higher, than the capacity, the first hospital may transfer part(s) of the protocol to another location with capacity. In this example, the first protocol step may be transferred to a second location.

The devices, protocol steps and staff requirements may be listed for the outputs required by the protocol and captured in an augmented protocol for the first location. However, more generally, the first protocol step can be compared to a second protocol step 408 at the second location to determine whether their outputs would be comparable in step 406.

The first and second protocol steps 402 and 408 may each be part of an augmented protocol at each location, where a plurality of comparable protocol steps are provided for each augmented protocol.

Comparability scores (e.g., similarities) between the protocol steps from the first and second locations may be used to rank the effort needed to modify the protocol step(s). In this example, the task of the first protocol step is to be performed at the second location to enable the continuity of the first protocol required at the first location. Thus, if required, the second protocol step is modified in step 410 such that the output of the modified protocol step is comparable to the output of the first protocol step. The scheduling of staff and devices at the second location can be arranged accordingly in step 412. Other staff expertise and devices might be required to accomplish the modified protocol step to better match the way of working of the first location.

Note that, in some cases, the second location may work differently from the first location for the same procedure steps. As such, the modifications in step 410 are done where needed to match the requirements of the first location. If the first and second protocol steps 402 and 408 are already comparable, no modification may be required.

Adaptations in scheduling and devices at the second location are carried out in step 412. A request for transfer of the protocol step is sent to the second location in step 414 together with the modified protocol step and the scheduling. The output from the modified protocol step, at the second location, is then sent to the first location.

The output may be a measurement performed at the second location. Alternatively, the output may be a report from data analysis performed at the second location. In general, the output is obtained by performing the second protocol step, or the modified protocol step, at the second location.

Optionally, the performance of the second location is determined in step 416. The output related performance assessment can be performed based on features calculated from the output. Output performance assessment can be calculated based on workflow data. For example, the workflow data may include information about performance of the steps prior to generation of the output, such as patient preparation, device and environment preparation, and device usage. The workflow data can also include information about the experience and expertise of the clinicians performing the operations. In addition, information about the capacity and demand in the performing location can also be included.

A performance score can be determined by checking whether pre-determined instructions set by (or agreed with) the first location were followed. For example, the match of device settings to the requirements set by the first location (e.g., in the modified protocol step) can be computed, and the match to workflow requirements can be computed.

The first location can decide to use the output provided by the second location and continue with the protocol at the first location, or can decide, based on the current (live) situation of the burden of the first location, to request another step in the protocol to be performed at a different location (e.g., at the second location).

In the use case above, the protocol step of the second location is adapted to be comparable to the protocol step in the first location. Another use case may be to transform protocol steps in the first location to match the second locations protocols. In this case, the first location can change the prior or consequent steps to make it possible that the transferred step can be performed at the second location, and that results obtained can be used, respectively.

Modifying the protocol steps may be advantageous as it is common that different locations use different protocol steps. This means that the comparability of outputs for a particular task, between different locations, cannot be guaranteed.

Hogeveen et al. did a comparison of international breast cancer guidelines and found out that the applicability for a hospital scoring was low. Applicability is whether the guidelines are clear and can easily be applied in a clinical setting. When guidelines from various organizations are unclear or conflicting, clinicians may become confused and frustrated. As a result, they may not apply the guidelines in their own practice (ref: 10.3747/co.19.930).

Embodiments of the current invention can check if comparable devices, staff and expertise are available to ensure high applicability.

Javier Pérez-Ardavin et al. "Comparison of three guidelines for screening, diagnosis and staging of prostate cancer in the USA and Europe" is a comparison of three guidelines for screening, diagnosis and staging of prostate cancer in the USA and Europe. Multiple differences between the guidelines were found in all stages. When transferring a protocol step, these differences in operations and interpretations should be included in the augmented protocols.

The decision to transport a critically ill patient, either within a hospital or to another facility, should be based on an assessment of the potential benefits of transport weighed against the potential risks.

Guidelines exist for the inter- and intrahospital transport of critically ill patients. Aspects of these guidelines can also be included in the augmented protocol to enable a proper and systematic way to do the transfer. Each step in the protocol can have a different benefit of transport weight against the potential risks.

In the case where there is more than one candidate healthcare provide (e.g., hospitals) which can be selected to perform measurements (or other outputs) required by the protocol applied in the first location, a ranking and selection mechanism can be considered, taking into account the disruption (or match) of using another hospital or location.

Such a system may include estimating the pressure/capacity at the first location, determining when the demand will exceed the capacity and determining the protocol steps which can be transferred to ease the pressure. Following a pre-determined criteria (e.g., certain radius from the current hospital), a list of candidate hospitals can be determined which can be utilized to help in execution of the protocol step. A second location is then selected. If arrangement with the second location is not successful, another location can be selected.

Another (later or previous) protocol step can be requested to be performed at the second location, even if the second location cannot perform the task of the first protocol step due to workflow/demand/capacity being changed at the first location, in which case the pool of potential candidate locations can again include the second location (where the assumption is that the second location rejected the specific first protocol step).

A use-case could be the response monitoring of a cancer treatment in which imaging is used multiple times a year to track progress or relapse. One or multiple of these imaging episodes could be performed, for example, in a second hospital and still allow data comparison of the previous imaging episodes performed by the original hospital.

Pancreatic cancer is a deadly cancer. Surgery is the only cure and, for that to work, early detection is important. Different specialties are involved in the care process, these include general practitioner, radiologist, pathologist, geneticist, HPB surgeon, gastroenterologist, anesthetist, oncologist, vascular surgeon, OR assistants and nurses.

The workflow for the pancreatic cancer care workflow can be divided in several stages:
1. Clinical presentation and diagnostics (location: peripheral hospital).
2. Diagnosis and treatment selection (location: expert hospital).
3. Treatment planning (location: expert hospital).
4. Surgical planning (location: expert hospital).
5. Resection surgery (location: expert hospital).
6. Discharge (location: expert hospital).
7. Systemic treatment (location: expert hospital).

The first stage of clinical presentation and diagnosis includes the steps:
- Anamnesis: medical history evaluation and physical examination.
- Radiological evaluation: diagnostic CT imaging tests, ultrasound, endoscopic ultrasound (EUS), endoscopic retrograde cholangiopancreatography (ERCP9) and, sometimes, MRI.
- Pancreatic imaging protocol: CT imaging protocol with contrast, MRI protocol with contrast and radiologist report.

Some of these steps can be requested by the peripheral hospital to be performed in the expert center, for example, in case of temporary insufficient staffing.

Some of these steps may be transferred to another hospital in case of high pressure in the original hospital. However, the transfer should be done in a meaningful manner so that the continuity of care is achieved, without having to repeat the measurements. For example, MRI imagining can be transferred to another location which follows a similar imaging protocol to the original hospital or where the protocol steps can be modified to achieve comparable outputs. In that way, a radiologist in the first hospital will be able to understand and interpret the imaging results obtained by the second hospital.

The second stage of diagnosis and treatment selection includes blood tests, tissue tests and a health condition analysis. The blood tests may include a complete blood count (CBC) measure for number of red blood cells, white blood cells and platelets, liver function tests for measuring liver made chemicals and a CA 19-9 test after biliary drainage, measured before and after neoadjuvant therapy and surgery.

The tissue tests may include EUS guided fine needle aspiration (FNA) biopsy and a tissue sample report. The health condition analysis is a check to understand what the general condition is and if patient can undergo treatments and surgery.

The second stage is executed in an expert hospital, which can be a different hospital than the hospital for the first stage. It can be the case, however, that some of the steps are performed in different expert or peripheral locations upon the requests and needs of the expert hospital. Transferring the protocol steps of the second stage can be achieved, with lower risk of repetition of the steps, by comparing the protocol steps between different locations as described above.

Other examples of protocols which can be transferred include pancreatic imaging protocols and pancreatic scope protocols.

Pancreatic imaging protocols may include the protocol steps including chest and pelvic CT with contrast, MRI (optional) - especially in case of indeterminate liver lesions suspected, PET (optional) - in case of high-risk patients or high-risk features such as image findings, elevated CA19-9, large primary tumors, large regional lymph nodes, excessive weight loss, extreme pain and an imaging report by a radiologist.

Pancreatic scope protocols may include the protocol steps of EUS inspection to guide biopsy of the pancreas, endoscopic retrograde cholangiopancreatography (ERCP) with contrast to take biopsies and stent placement.

Protocol steps may be repeated several times at a later time (depending on new findings, patient complaints, requests by radiologists, change in measurement devices, etc.). In some cases, when the capacity of the expert hospital is insufficient, they may need to be performed in another location. However, the selection of this location becomes critical, and needs to be done so that the measurements performed in the secondary location can be used by the primary location. For this, the approach of labelling the measurements related steps with labels linked to device settings, device inputs, device outputs, patient preparation, and staff expertise indicators becomes critical.

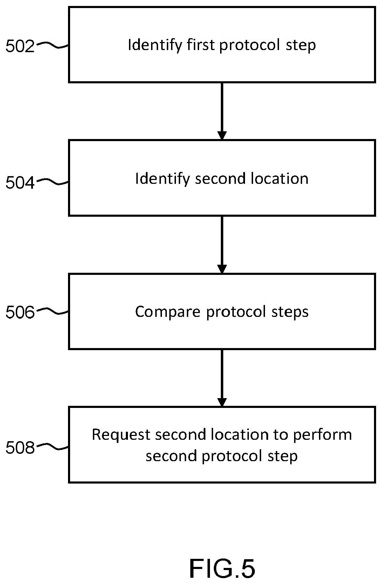

Fig. 5 shows a method for requesting a protocol step to be performed at a different medical location. The method comprises identifying, at a first location, a first protocol step to be performed on a subject in step 502. Based on identifying the first protocol step to be performed on the subject, a second location with a second protocol step is identified in step 504 and the first protocol step and the second protocol step are compared to determine whether a first output from performing the first protocol step would be comparable to a second output from performing the second protocol step in step 506. Comparing the protocol steps may include comparing devices specified by the protocol steps (e.g., comparing specifications of the devices), comparing software used by the protocol steps, comparing parameters (for the devices and/or software) specified by the protocol steps and/or comparing previous outputs obtained via the protocol steps.

The second location is requested to perform the second protocol step on the subject based on the comparison. For example, if the comparison determines that the outputs would be comparable, the second location is requested to perform the second protocol step. If the comparison determines that the outputs would not be comparable, the second location may be requested to perform the modified protocol step which provides a third output which is comparable to the first output.

A processor in a system may be used to perform all of the steps in the method shown in Fig. 5.

The skilled person would be readily capable of developing a processor for carrying out any herein described method. Thus, each step of a flow chart may represent a different action performed by a processor and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method for requesting a protocol step to be performed at a different medical location, the method comprising:
identifying (502), at a first location, a first protocol step to be performed; and
based on identifying the first protocol step to be performed:
identifying (504) a second location with a second protocol step;
comparing (506) the first protocol step and the second protocol step to determine whether a first output from performing the first protocol step would be comparable to a second output from performing the second protocol step; and
based on the comparison, requesting (508) the second location to perform the second protocol step.

2. The method of claim 1, wherein requesting the second location to perform the second protocol step comprises:
based on the first output being determined to not be comparable to the second output, modifying the second protocol step based on the first protocol step to generate a modified protocol step, such that the first output would be comparable to a third output of the modified protocol step; and
requesting the second location to perform the modified protocol step.

3. The method of claims 1 or 2, wherein comparing the first protocol step and the second protocol step comprises:
identifying a plurality of comparable protocol steps, wherein an output of each of the comparable protocol steps is comparable to the first output; and
comparing the second protocol steps and the comparable protocol steps.

4. The method of claims 2 and 3, wherein modifying the second protocol step comprises adapting the second protocol step based on the one or more comparable protocol steps.

5. The method of claims 3 or 4, wherein identifying the plurality of comparable protocol steps comprises:
identifying a plurality of medical resources at different medical locations capable of generating an output related to the first protocol step;
obtaining parameters for the identified medical resources;
determining whether the identified medical resources, using the corresponding parameters, are capable of generating an output comparable to the first output; and
identifying comparable protocol steps, each indicating a medical resource and the corresponding parameters, based on the identified medical resources and the corresponding parameters which are capable of generating an output comparable to the first output.

6. The method of claim 5, wherein identifying the plurality of comparable protocol steps further comprises determining a capacity of the different medical locations corresponding to the identified medical resources, wherein identifying comparable protocol steps is further based on the capacity of the different medical locations.

7. The method of any of claims 1 to 6, wherein:
the first protocol step indicates a first medical resource capable of generating the first output and one or more first parameters of the first medical resource suitable for generating the first output,
the second protocol step indicates a second medical resource capable of generating the second output and one or more second parameters of the second medical resource, and
comparing the first protocol step and the second protocol comprises comparing the first medical resource and the second medical resources and comparing the first parameters and the second parameters.

8. The method of any of claims 1 to 7, further comprising determining a capacity of the first location and/or second location, wherein requesting the second location to perform the second protocol step is based on the capacity of the first location and/or the second location.

9. The method of claim 8, wherein the capacity of the first location and/or the second location includes one or more of:
the availability of staff capable of performing the first protocol step or the second protocol step respectively; and
the availability of medical resources required for the first protocol step or the second protocol step respectively.

10. The method of any of claims 1 to 9, wherein the first and second outputs are imaging measurements on a subject.

11. The method of claim 10, further comprising imaging the subject, using the second protocol step, at the second location.

12. The method of any of claims 1 to 11, further comprising adapting one or more later, or previous, protocol steps to be performed by the first location based on the second output of the second protocol step.

13. A computer program carrier comprising computer program code which, when executed on a processing system, causes the processing system to perform all of the steps of the method according to any of the preceding claims.

14. A system for requesting a protocol step to be performed at a different medical location, the system comprising a processor configured to:
identify (502), at a first location, a first protocol step to be performed; and
based on identifying the first protocol step to be performed:
identify (504) a second location with a second protocol step;
compare (506) the first protocol step and the second protocol step to determine whether a first output from performing the first protocol step would be comparable to a second output from performing the second protocol step; and
based on the comparison, request (508) the second location to perform the second protocol step.

15. The system of claim 14, wherein the processor is configured to request the second location to perform the second protocol step by:
based on the first output being determined to not be comparable to the second output, modifying the second protocol step based on the first protocol step to generate a modified protocol step, such that the first output would be comparable to a third output of the modified protocol step; and
requesting the second location to perform the modified protocol step.
